## Europäisches Patentamt
## European Patent Office
## Office européen des brevets

(19)

(11) Publication number: **0 004 105**
**B1**

# EUROPEAN PATENT SPECIFICATION

(12)

(45) Date of publication of patent specification: **12.08.81**

(51) Int. Cl.³: **C 07 C 27/12**

(21) Application number: **79200086.1**

(22) Date of filing: **20.02.79**

(54) Process for preparing cycloalkanols and cycloalkanones.

(30) Priority: **25.02.78 NL 7802125**

(43) Date of publication of application:
**19.09.79 Bulletin 79/19**

(45) Publication of the grant of the European patent:
**12.08.81 Bulletin 81/32**

(84) Designated Contracting States:
**BE DE FR GB IT NL**

(56) References cited:
**DE - A - 2 148 322**
**FR - A - 2 070 977**
**FR - A - 2 208 868**
**GB - A - 1 382 849**

(73) Proprietor: **STAMICARBON B.V.**
**Postbus 10**
**NL-6160 MC Geleen (NL)**

(72) Inventor: **Bryan, William Ole**
**Dunantstraat 14**
**Geleen (NL)**

(74) Representative: **Hoogstraten, Willem Cornelis**
**Roeland et al,**
**OCTROOIBUREAU DSM Postbus 9**
**NL-6160 MA Geleen (NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Process for preparing cycloalkanols and cycloalkanones

The invention relates to a process for preparing cycloalkanols and cycloalkanones by oxidation in the liquid phase of a cycloalkane having 5 to 12 carbon atoms in the ring by means of a gas containing molecular oxygen, thus obtaining an oxidation mixture containing cycloalkyl hydroperoxide which oxidation mixture is subsequently treated, in the presence of an aqueous solution of an alkali metal hydroxide, with a metal salt that causes decomposition of the cycloalkyl hydroperoxide. This type of process is known from the British patent specification No. 1.382.849.

In the known process the oxidation mixture is treated with the alkaline solution simultaneously with or after the addition of the cycloalkyl hydroperoxide-decomposing metal salt, while the pH of the aqueous phase in the reaction mixture is kept between 8 and 13, measured at 25°C. The drawback of this process is that the yield of cycloalkanol and cycloalkanone based on cycloalkane converted is low. The applicant has found that this output can be enhanced by setting the pH of the aqueous phase considerably higher, for instance corresponding with 0.5 moles/l of free sodium hydroxide or even more, but this means a very high consumption of alkali, which affects the process economically.

The applicant has found that a high yield of cycloalkanol and cycloalkanone can be reached at a low alkali consumption by neutralizing the acids present as by-products in the oxidation mixture before treating the mixture with the cycloalkyl hydroperoxide-decomposing metal salt in the presence of the alkaline solution.

It is pointed out that in the French patents 2070977 and 2208868 the possibility is mentioned to subject the oxidation mixture to a water washing. This does not result in a neutralization of this mixture.

According to the invention, therefore, cycloalkanols and cycloalkanones are prepared by oxidation in the liquid phase of a cycloalkane with 5 to 12 carbon atoms in the ring by means of a gas containing molecular oxygen, thus obtaining an oxidation mixture containing cycloalkyl hydroperoxide, which oxidation mixture in a subsequent step b) is treated, in the presence of an aqueous solution of an alkali metal hydroxide, with a metal salt that causes decomposition of the cycloalkyl hydroperoxide, characterized in that, prior to the treatment with the metal salt in step b), the acids present in the oxidation mixture are neutralized in a step a).

The process starts from a cycloalkane with 5 to 12 carbon atoms in the ring, in particular cyclopentane, cyclododecane and especially cyclohexane. The invention will be elucidated in particular with reference to the preparation of cyclohexanol and cyclohexanone from cyclohexane. Besides the cycloalkyl hydroperoxide, the oxidation mixture contains minor quantities of other peroxides, such as the corresponding ω-hydroperoxyalkane carboxylic acid and dicycloalkylperoxide, which for the sake of simplicity are included under the term "cycloalkyl hydroperoxide" herein. The oxidation takes place in the liquid phase. As molecular-oxygen-containing gas, air or pure oxygen can be used, for instance, or nitrogen-oxygen mixtures of a composition differing from that of air, such as mixtures of air and reactor offgas. The process features low conversion rates relative to the cycloalkane supplied, from 1 to 12% for instance. Suitable oxidation temperatures range between 120 and 200°C; preferably, the operating temperature lies between 140 and 180°C. The operating pressure is not critical, but should be such that a liquid phase is maintained in the system. The pressure mostly lies between 400 and 5000 kPa, for instance at about 1300 kPa.

Preferably, the oxidation reaction is carried out in the absence of any substances which will promote the decomposition of cycloalkyl hydroperoxide, such as compounds of transition metals. To effect this object, use can be made of reactors with an inert inner wall made of, for instance, passivated steel, aluminium, tantalium, glass, enamel and the like. In this manner the undesired decomposition of the cycloalkyl hydroperoxide under the oxidation conditions is avoided as much as possible. If desired, however, small quantities of oxidation catalysts may be present, for instance not more than 10 ppm and preferably not more than 1 ppm of a transition metal in the form of a soluble salt. The transition metal is cobalt by preference, but may also be chromium, manganese, iron, nickel or copper, for instance. Cobalt naphthenate or cobalt 2-ethylhexanoate is very suitable.

The oxidation reaction yields a pressurized, hot, rather diluted solution of cycloalkyl hydroperoxide in cycloalkane. It is efficient to allow subsequently the solution obtained to expand to a lower pressure, for instance to about 1000 kPa.

Next, the acids present in the oxidation mixture are neutralized (step a). This can be done by addition of an aqueous solution of a hydroxide or carbonate of an alkali metal. Preferably such a quantity of base is added that the pH of the water layer of the product mixture is higher than 7 at 25°C, preferably 8 to 13. Suitable bases are for instance sodium hydroxide, sodium carbonate, potassium hydroxide and potassium carbonate. An advantage of the process according to the invention is that in the neutralization, which involves the largest consumption of base, alkali metal carbonate can be used as base and there is no need for a costly alkali metal hydroxide.

In a preferred embodiment of the process according to the invention, the alkaline solution used in the neutralization is comprised at least in part of at least a portion of the water phase obtained from the peroxide decomposition treatment described hereafter (step b). In this manner the alkali consumption can be reduced further.

In another preferred embodiment, which can be combined with the preceding one, the alkaline solution used in the neutralization is an aqueous solution of alkali metal carbonate obtained through combustion, known in itself, of the effluent which is obtained from the preparation of cycloalkanones and cycloalkanols through oxidation of cycloalkanes and contains alkali metal salts of carboxylic acids (see for instance the British patent specification No. 1.398.293). In the process according to the invention, such effluent is formed in step a) as well as in step b). This measure leads to additional considerable reduction of the alkali consumption.

The neutralization takes place at a temperature of for instance 80—170°C, preferably of 130—160°C.

After the neutralization (step a) the aqueous phase is preferably separated off before proceeding to the peroxide decomposition treatment with the cycloalkyl hydroperoxide-decomposing metal salt in the presence of an aqueous solution of an alkali metal hydroxide (step b). If desired, the oxidation mixture may be washed with water prior to the peroxide decomposition treatment.

In the peroxide decomposition treatment such a quantity of alkali metal hydroxide is preferably used that the $OH^-$ concentration of the water layer upon completion of the decomposition is at least 0.1 moles/l, preferably at least 0.6 moles/l. An $OH^-$ concentration higher than 2 moles/l offers no advantage, but may be used. In practice, 0.6—1 moles/l is most suitable. Suitable alkali metal hydroxides are for instance sodium hydroxide and potassium hydroxide.

The cycloalkyl hydroperoxide-decomposing metal salt normally is a salt of a transition metal, preferably cobalt, although chromium, manganese, iron nickel or copper, for instance, may also be used. Preferably the metal salt is water soluble. The sulphates and acetates are very suitable. The quantity of metal salt is for instance 0.1 to 1000 ppm by weight, calculated as the metal relative to the weight of the water phase. Larger quantities of metal salt may be used, but offer no advantage. Preferably 0.1 to 10 ppm of metal is used. The metal salt can be added efficiently to the reaction mixture in the form of an aqueous solution, possibly together with the alkali metal hydroxide. It is also possible to add the metal to the reaction mixture in the form of an organic salt dissolved in an organic solvent, for instance the cycloalkane in question.

In order to effect an efficient peroxide decomposition, the volume ratio between the aqueous phase and the organic phase in the decomposition reactor is preferably kept at at least about 0.02, preferably at 0.05—0.20. Higher volume ratios may be used, but offer no particular advantage.

The hydroperoxide decomposition takes place at a temperature of for instance 80—170°C.

After completion of the decomposition reaction, the resultant aqueous layer can be separated off from the reaction mixture, the reaction mixture can be washed with water to remove salt residue, if desired, and the cyclohalkanol and the cycloalkanone can be isolated by means of distillation. The non-converted cycloalkane can be returned to the oxidation, the water phase to the neutralization.

The process according to the invention lends itself for batch production as well as continuous production.

The invention will now be elucidated with the aid of the following examples and the comparative experiment.

Example 1

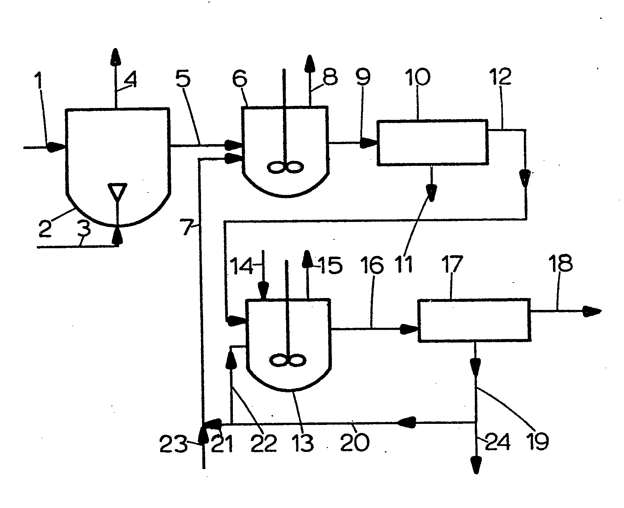

Reference is made to the attached figure, representing a possible reaction diagram of the process according to the invention. Lines 22, 23, 24 are not in use in this example.

In oxidation reactor 2 cyclohexane supplied through lines 1, is oxidized in the liquid phase, with air supplied through line 3, at a temperature of 165°C and a pressure of 1050 kPa, in the absence of a metal salt as catalyst. The rate of conversion is 4.5 mol.%. The oxidation mixture contains 250 mmol of peroxide (calculated as cyclohexyl hydroperoxide) per kg. From the offgas, discharged through line 4, cyclohexane is recovered in the usual manner and returned to the oxidation reaction, after which the residual gas is blown off.

The oxidation mixture is supplied through line 5 to neutralization vessel 6, in which it is cooled through expansion to 145°C and the acids in the mixture are neutralized with effluent supplied through line 7 from the phase separator after the decomposition reactor. Inert gas is not supplied to vessel 6. The offgas discharged through line 8 is condensed, separated into an aqueous phase, which is discharged, and an organic phase, which is returned as cyclohexane feed to oxidation reactor 2. The condensation heat can be used elsewhere in the process.

After the neutralization the liquid reaction mixture is supplied through line 9 to separator 10, where the two liquid phases are separated. The aqueous phase is discharged through line 11 as waste water, from which sodium carbonate can be obtained through combustion. The organic phase is supplied through line 12 to decomposition reactor 13, where it is thoroughly mixed with 5% by vol. of an aqueous solution of sodium hydroxide which contains 1 ppm $CoSO_4$ and is supplied through line 14. The concentration of the sodium hydroxide is so chosen that the hydroxyl ion concentration in the aqueous phase discharged through line 11 and obtained in the neutralization, is 0.01 moles/l. The gas phase discharged through line 15 is treated as described for the gas phase in line 8. Through line 16 the liquid reaction mixture goes to separator 17, where it is separated into an aqueous phase, which goes through lines 19, 20, 21 and 7 to neutralization vessel 6, and an organic phase, which is discharged through line 18 and processed into cyclohexanone and cyclohexanol through distillation by usual methods. The non-converted cyclohexane recovered through this distillation is returned to oxidation reactor 2.

The cyclohexyl hydroperoxide is converted for 96%. The efficiency of the conversion to cyclohexanol plus cyclohexanone amounts to 94%. The quantity of free sodium hydroxide in the aqueous effluent is negligible.

### Example II

Operation is in accordance with the diagram shown in the attached figure. Line 21 is not in use.

In neutralization vessel 6, an oxidation mixture obtained as in example I is cooled through expansion to 145°C and neutralized with such an excess of sodium carbonate that the pH of the aqueous phase discharged through line 11 amounts to 10. The sodium carbonate is supplied to neutralization vessel 6 through lines 23 and 7 in the form of an aqueous solution in a quantity of at least 5% by vol. relative to the oxidation mixture. After separation of the phases in separator 10, the organic phase is supplied to decomposition reactor 13, which is also supplied with an aqueous sodium hydroxide solution containing 1 ppm $CoSO_4$ through line 14.

In this embodiment of the process according to the invention the hydroxide consumption is very low. Maintaining a volume ratio of about 0.05 between the aqueous phase and the organic phase in the decomposition reactor, the hydroxide should therefore have to be supplied as a highly diluted solution, which is less practical. This problem is solved by returning such a part of the aqueous effluent from separator 17 to decomposition reactor 13 through lines 19, 20 and 22 as to effect said ratio. The sodium hydroxide concentration in line 14 is in the order of 20% by wt., but is so controlled that the hydroxyl ion concentration in the aqueous phase discharged through line 19 amounts to 1 mole/.l.

The cyclohexyl hydroperoxide is converted for 96%. The efficiency of conversion to cyclohexanol and cyclohexanone amounts to 94%.

The part of the flow in line 19 that is not returned to decomposition reactor 13 is in this example discharged through line 24. This effluent stream is relatively small and so the loss of hydroxide is low. Base consumption can be further reduced by leading this flow to neutralization vessel 6 through lines 21 and 7.

The aqueous effluents from lines 11 and 24 can be combusted in the known manner, giving sodium carbonate and/or sodium hydroxide. In advantageous embodiment of the process according to the invention the conditions of the combustion are in known manner so chosen that the solid product mainly consists of sodium carbonate; this product is dissolved in water and the solution is supplied to neutralization vessel 6 through lines 23 and 7.

### Comparative Example A

An oxidation mixture obtained in accordance with example I is thoroughly mixed at 165°C with 5% by vol. of an aqueous sodium hydroxide solution, containing 1 ppm $CoSO_4$, in a continuous-flow ideally mixing reactor. The concentration of the sodium hydroxide has been so chosen that the hydroxyl ion concentration in the aqueous phase after separation is 0.1 moles/l.

The cyclohexyl hydroperoxide has been converted to only 65% and the efficiency of conversion to cyclohexanol plus cyclohexanone is 87%.

### Comparative Experiment B

Comparative example A is repeated, but now the concentration of the sodium hydroxide has been so chosen that the hydroxyl ion concentration in the aqueous phase after phase separation is 0.6 moles/l.

The cyclohexyl hydroperoxide has now been converted to 96% and the efficiency of conversion to cyclohexanol and cyclohexanone is 94%.

The quantity of free sodium hydroxide in the aqueous effluent, however, corresponds with a quantity of 36 kg NaOH/tonne cyclohexanone. This affects the economic attractiveness of the process.

**Claims**

1. Process for preparing cycloalkanols and cycloalkanones by oxidation in the liquid phase of a cycloalkane with 5 to 12 carbon atoms in the ring by means of a gas containing molecular oxygen, thus obtaining an oxidation mixture containing cycloalkyl hydroperoxide, which oxidation mixture in a step b) is treated, in the presence of an aqueous solution of an alkali metal hydroxide, with a metal salt that causes decomposition of the cycloalkyl hydroperoxide, characterized in that, prior to the treatment with the metal salt in step b), the acids present in the oxidation mixture are partly or wholly neutralized in a step a).

2. Process according to claim 1, characterized in that in step a) the acids are neutralized by addition of an aqueous solution of an alkali metal hydroxide or carbonate.

3. Process according to claim 2, characterized in that in step a) such a quantity of alkali metal hydroxide or carbonate solution is added that the pH of the water layer of the product mixture of the neutralization is 8 to 13 at 25°C.

4. Process according to any one of the claims 1—3, characterized in that in step a) the acids are neutralized by addition of a neutralising agent comprized at least in part of at least a portion of the aqueous phase obtained in step b).

5. Process according to any one of the claims 2—4, characterized in that in step a) the acids are neutralized by addition of an aqueous solution of an alkali metal carbonate obtained by means of combustion of the effluent arising from step a) and/or step b).

6. Process according to any one of the claims 1—5, characterized in that after the neutralization in step a) the aqueous phase is separated off before proceeding to step b).

7. Process according to claim 6, characterized in that after the neutralization in step a) the product mixture is washed with water before proceeding to step b).

8. Process according to any one of the claims 1—7, characterized in that in step b) in the treatment with the cycloalkyl hydroperoxide-decomposing metal salt such a quantity of alkali metal hydroxide is added that the OH$^-$ concentration in the water layer of the product mixture is 0.1—2 moles/l.

9. Process according to any one of the claims 1—8, characterized in that the metal salt used is a water-soluble transition metal salt in a quantity of 0.1 to 1000 ppm, calculated as metal relative to the weight of the water phase.

10. Process according to any one of the claims 1—9 characterized in that the volume ratio between the aqueous phase and the organic phase in the decomposition reactor is at least 0.02.

**Revendications**

1. Procédé pour la préparation de cycloalcanols et de cycloalcanones par oxydation dans la phase liquide d'un cycloalcane ayant de 5 à 12 atomes de carbone dans le cycle au moyen d'un gaz contenant de l'oxygène moléculaire ce qui donne un mélange d'oxydation contenant un hydroperoxyde de cycloalcoyle, lequel mélange d'oxydation dans une étape b) est traité, en présence d'une solution aqueuse d'un hydroxyde de métal alcalin, par un sel de métal qui cause une décomposition de l'hydroperoxyde de cycloalcoyle, caractérisé en ce que, avant le traitement par le sel de métal dans l'étape b), les acides présents dans le mélange d'oxydation sont partiellement ou complètement neutralisés dans une étape a).

2. Procédé selon la revendication 1, caractérisé en ce que dans l'étape a) les acides sont neutralisés par addition d'une solution aqueuse d'un hydroxyde ou carbonate de métal alcalin.

3. Procédé selon la revendication 2, caractérisé en ce que dans l'étape (a) on ajoute une quantité de solution d'hydroxyde ou de carbonate de métal alcalin telle que le pH de la couche aqueuse du mélange de produits de la neutralisation soit compris entre 8 et 13 à 25°C.

4. Procédé selon l'une quelconque des revendications 1 à 3, caractérisé en ce que dans l'étape a) les acides sont neutralisés par addition d'un agent de neutralisation constitué au moins en partie d'au moins une portion de la phase aqueuse obtenue dans l'étape b).

5. Procédé selon l'une quelconque des revendications 2 à 4, caractérisé en ce que dans l'étape a) les acides sont neutralisés par addition d'une solution aqueuse d'un carbonate de métal alcalin obtenu par combustion de l'effluent résultant de l'étape a) et/ou de l'étape b).

6. Procédé selon l'une quelconque des revendications 1 à 5, caractérisé en ce qu'après la neutralisation dans l'étape a) on sépare la phase aqueuse avant de procéder à l'étape b).

7. Procédé selon la revendication 6, caractérisé en ce qu'après la neutralisation dans l'étape a), le mélange de produits est lavé à l'eau avant exécution de l'étape b).

8. Procédé selon l'une quelconque des revendications 1 à 7, caractérisé en ce que dans l'étape b) dans le traitement par le sel de métal décomposant l'hydroperoxyde de cycloalcoyle, on ajoute une quantité d'hydroxyde de métal alcalin telle que la concentration des ions OH$^-$ dans la couche aqueuse du mélange de produits soit de 0,1 à 2 moles par litre.

9. Procédé selon l'une quelconque des revendications 1 à 8, caractérisé en ce que le sel de métal utilisé est un sel de métal de transition soluble dans l'eau dans une quantité de 0,1 à 1000 ppm, en calculant en métal par rapport au poids de la phase aqueuse.

10. Procédé selon l'une quelconque des revendications 1 à 9, caractérisé en ce que le rapport en volume entre la phase aqueuse et la phase organique dans le réacteur de décomposition est d'au moins 0,02.

**Patentansprüche**

1. Verfahren zum Herstellen von Cycloalkanolen und Cycloalkanonen durch Oxidieren eines Cycloalkans mit 5 bis 12 Kohlenstoffatomen im Ring in der Flüssigphase mit eineme Molekularsauerstoff enthaltenden Gas zu einem Cycloalkylhydroperoxyd haltigen Oxidationsgemisch, wonach man in einer Stuf b) das Oxidationsgemisch mit einem Cycloalkylhydroperoxyd zersetzenden Metallsalz in Anwesenheit einer wässrigen Lösung eines Alkalimetallhydroxyds behandelt, dadurch gekennzeichnet, dass man für das Behandeln des Metallsalzes in der Stufe b) in einer Stufe a) die im Oxidationsgemisch enthaltenden Säuren ganz oder teilweise neutralisiert.

2. Verfahren nach Anspruch 1, dadurch gekennzeichnet, dass man in der Stufe a) die Säuren durch Hinzufügen einer wässrigen Alkalimetallhydroxyd- oder -carbonatlösung neutralisiert.

3. Verfahren nach Anspruch 2, dadurch gekennzeichnet, dass man in der Stufe a) soviel Alkalimetallhydroxyd- oder -carbonatlösung bei-gibt, dass der pH-Wert der Wasserschicht des Produktgemisches der Neutralisation 8 bis 13 bei 25 °C beträgt.

4. Verfahren nach einem der Ansprüche 1 bis 3, dadurch gekennzeichnet, dass man in der Stufe a) die Säuren durch Zusetzen eines neutralisierenden Agens neutralisiert, das wenigstens teilweise aus jedenfalls einem Teil der bei der Stufe b) anfallenden Wasserphase besteht.

5. Verfahren nach einem der Ansprüche 2 bis 4, dadurch gekennzeichnet, dass man in der Stufe a) die Säuren durch Biegeben einer wäss-rigen Lösung von Alkalimetallcarbonat neutral-isiert, das durch Verbrennen des in der Stufe a) und/oder b) anfallenden Abwassers anfällt.

6. Verfahren nach einem der Ansprüche 1 bis 5, dadurch gekennzeichnet, dass man nach der Neutralisation in der Stufe a) die wässrige Phase abscheidet, ehe man zur Stufe b) übergeht.

7. Verfahren nach Anspruch 6, dadurch gekennzeichnet, dass man nach der Neutralisation in der Stufe a) das Produktgemisch mit Wasser auswäscht, ehe man zur Stufe b) übergeht.

8. Verfahren nach einem der Ansprüche 1 bis 7, dadurch gekennzeichnet, dass man in der Stufe b) beim Behandeln mit dem Cycloalkylhydroperoxyd zersetzenden Melallsalz soviel Alkalimetallhydroxyd hinzufügt, dass die OH$^-$-Konzentration in der Wasserschicht des Produktgemisches 0,1 bis 2 N beträgt.

9. Verfahren nach einem der Ansprüche 1 bis 8, dadurch gekennzeichnet, dass man als Metallsalz ein wasserlösliches Übergangsmetallsalz in einer Menge von 0,1 bis 1000 Teile pro Million, berechnet als Metall, bezogen auf das Gewicht der Wasserphase, verwendet.

10. Verfahren nach einem der Ansprüche 1 bis 9, dadurch gekennzeichnet, dass das Volumenverhältnis wässrige Phase: organische Phase im Zersetzungsreaktor mindestens 0.02 beträgt.